(19) Europäisches Patentamt
European Patent Office
Office européen des brevets

(11) Veröffentlichungsnummer: **0 291 806 B1**

(12) # EUROPÄISCHE PATENTSCHRIFT

(45) Veröffentlichungstag der Patentschrift: **26.08.92**

(51) Int. Cl.$^5$: **C07C 45/60**, C07C 47/198, C07C 47/277, C07D 335/02

(21) Anmeldenummer: **88107403.3**

(22) Anmeldetag: **07.05.88**

(54) **Umwandlung von 1,3-Dioxanen zu 4-Oxaaldehyden.**

(30) Priorität: **12.05.87 DE 3715755**

(43) Veröffentlichungstag der Anmeldung:
**23.11.88 Patentblatt 88/47**

(45) Bekanntmachung des Hinweises auf die Patenterteilung:
**26.08.92 Patentblatt 92/35**

(84) Benannte Vertragsstaaten:
**BE DE FR GB NL**

(56) Entgegenhaltungen:
**EP-A- 0 199 210**
**DE-A- 2 922 698**
**US-A- 3 676 500**

**JOURNAL OF THE AMERICAN CHEMICAL SOCIETY, Band 84, 5. September 1962, Seiten 3307-3319; C.S. RONDESTVEDT, Jr. et al.: "A new rearrangement. Catalytic isomerization of m-dioxanes to beta-alkoxy aldehydes. II. Scope and limitations"**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18c**
**W-6710 Frankenthal(DE)**
Erfinder: **Merger, Franz, Dr.**
**Max-Slevogt-Strasse 25**
**W-6710 Frankenthal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Anmeldung betrifft ein Verfahren zur Herstellung von 4-Oxa-aldehyden durch katalytische Isomerisierung von 1,3-Dioxanen.

Es ist bekannt, daß 1,3-Dioxan und dessen Derivate zu β-Alkoxyaldehyden umgelagert werden können (J. Am. Chem. Soc. 82 (1960), 6419 und J. Am. Chem. Soc. 84 (1962), 3307). Als Katalysatoren wurden hierbei Silicagel und Bimsstein eingesetzt. Die verwendeten Katalysatoren zeigen starke Desaktivierungserscheinungen. Auch sind ihre Aktivität und Selektivität wirtschaftlich noch nicht befriedigend. Wegen der unzureichenden Spezifizierung des Naturproduktes Bimsstein, das je nach Herkunft unterschiedliche Zusammensetzung aufweisen kann, sind unkontrollierbare Einflüsse auf die Reaktion nicht zu vermeiden, (Houben Weyl, Methoden d. org. Chemie IV, 2, S. 149 (1955)).

In der DE-OS 29 22 698 wird ein Verfahren zur Herstellung von β-Alkoxypivalinaldehyd aus 1,3-Dioxanen unter Verwendung von Siliciumdioxid, dotiert mit Hydroxiden der Gruppe III A und/oder III B und Alkalihydroxid, als Katalysator beschrieben. Diese Katalysatoren, die aber nur einen graduellen Fortschritt bringen, unterscheiden sich von den vorher beschriebenen durch Neutralisation der aciden Zentren. Die für die Katalysatorfertigung notwendigen Verbindungen der reinen Lanthanide Praseodym und Neodym sind teure und nicht leicht zugängliche Chemikalien. Das bevorzugt benutzte handelsübliche Lanthaniden-Gemisch Didymium variiert in seiner Zusammensetzung, so daß die Reproduzierung der technischen Katalysatoren schwierig ist. Die angegebenen Katalysatorstandzeiten liegen nur im Stundenbereich; über die Regenerierung der Katalysatoren wird keine Aussage gemacht.

Der Erfindung liegt die Aufgabe zugrunde, ein Verfahren zu entwickeln, 4-Oxa-aldehyde aus den entsprechenden 1,3-Dioxanen herzustellen in Gegenwart von Katalysatoren, die sich durch einfache und kostengünstige Verfügbarkeit, hohe Aktivität und leichte Regenerierbarkeit auszeichnen. Weiterhin sollen bei langen Katalysatorstandzeiten hohe Umsätze und Selektivitäten gewährleistet sein.

Gegenstand der Erfindung ist ein Verfahren zur Herstellung von 4-Oxa-aldehyden der Formel (I)

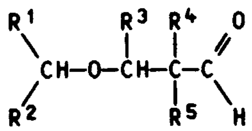

(I)

durch katalytische Isomerisierung von 1,3-Dioxanen, bei dem man 1,3-Dioxane der Formel (II)

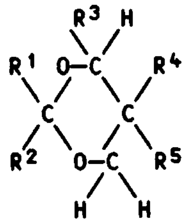

(II),

wobei die Reste $R^1$, $R^2$, $R^4$ und $R^5$ in den Formeln (I) und (II) untereinander gleich oder verschieden sind und für Wasserstoff, geradkettige oder verzweigte Alkyl-, Alkenyl oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl- oder Alkenylarylreste mit 6 bis 16 Kohlenstoffatomen oder für heterocyclische Reste stehen und darüber hinaus jeweils die Reste $R^1$ und $R^2$ und/oder $R^4$ und $R^5$ zusammen mit dem C-Atom an das sie gebunden sind, ein Cycloalkan, Cycloalken oder einen Heterocyclus mit 5 bis 7 Ringgliedern bilden können, wobei die genannten Reste noch unter den Reaktionsbedingungen inerte Substituenten tragen können und wobei der Rest $R^3$ Wasserstoff oder einen geradkettigen oder verzweigten Alkylrest bedeutet, unter Verwendung von Metalloxid-Katalysatoren, ohne Zeolithstruktur, die mit anorganischen Säuren behandelt worden sind, isomerisiert.

Bei dem erfindungsgemäßen Verfahren werden die eingangs an die Katalysatoren gestellten Anforderungen weitgehend erfüllt. Im Hinblick auf die Lehren des Standes der Technik ist das Ergebnis des Verfahrens besonders überraschend, weil die Lehre gerade in entgegengesetzte Richtung, nämlich Ausschaltung der aciden Zentren, wies. Man konnte daher nicht erwarten, daß gerade mit sauren Metalloxid-Katalysatoren die sich durch hohe Acidität in weiten Grenzen hervorragende Ergebnisse erhalten werden.

Die Umwandlung der 1,3-Dioxane zu 4-Oxa-aldehyden ist eine gute Methode, um beispielsweise die Ether der Hydroxyneoalkanale in hoher Selektivität bei hohem Umsatz herzustellen, die nach konventionellen Veretherungsmethoden nicht bzw. nur schwierig zugänglich sind.

Die Umwandlung läßt sich durch folgende Gleichung wiedergeben:

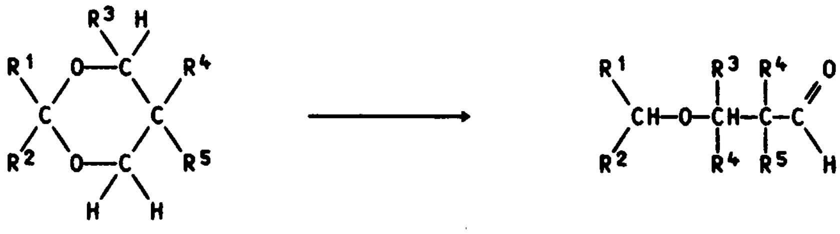

(II) (I)

Die als Ausgangsstoffe verwendeten 1,3-Dioxane der Formel (II) und dementsprechend die entstehenden 4-Oxa-aldehyde der Formel (I) enthalten die Reste $R^1$, $R^2$, $R^4$ und $R^5$, die gleich oder verschieden sind und unabhängig voneinander für Wasserstoff, verzweigte oder unverzweigte, Alkyl-, Alkenyl- oder Alkinylreste mit bis zu 18 Kohlenstoffatomen, insbesondere 1 bis 12, vorzugsweise 1 bis 6 Kohlenstoffatomen, Cycloalkyl-oder Cycloalkenylreste mit 5 bis 8, insbesondere 5 und 6 Kohlenstoffatome, Aryl-,Alkylaryl-, Aralkenyl- oder Alkenylarylreste mit 6 bis 16, insbesondere 6 bis 12 Kohlenstoffatomen, aromatische gesättigte und ungesättigte Heterocyclen, die ein oder mehrere Heteroatome, wie Stickstoff, Sauerstoff oder Schwefel enthalten. Die genannten Reste können noch unter den Reaktionsbedingungen inerte Substituenten tragen.

Die aus Verbindung (II) entstandenen Produkte (I) können ihrerseits mit einem Diol über ein Acetal der allgemeinen Formel (IV)

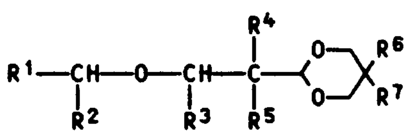

(IV),

in der die Reste $R^6$ und $R^7$ im oben angegebenen Definitionsbereich von $R^4$ und $R^5$ liegen, zu Aldehyden der Struktur (Formel III)

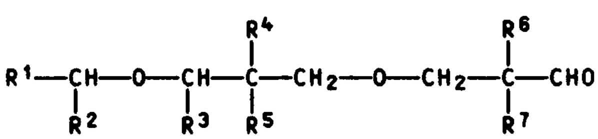

(III)

weiter aufgebaut werden.

Die Reste $R^1$ und $R^2$ und/oder $R^4$ und $R^5$ und/oder $R^6$ und $R^7$ können auch zusammen mit dem C-Atom, an das sie gebunden sind, ein Cycloalkan, Cycloalken oder einen Heterocyclus bilden. Als Rest $R^3$ kommen unabhängig von den übrigen Resten Wasserstoff sowie geradkettige oder verzweigte Alkylreste mit 1 bis 12, insbesondere 1 bis 8, vorzugsweise 1 bis 4 Kohlenstoffatomen in Betracht.

Alkyl-, Alkenyl- oder Alkinylreste sind z.B. Methyl-, Ethyl-, n-Propyl-, i-Propyl-, Propenyl-, i-Propenyl-, n-Butyl-, i-Butyl-, n-Butenyl-, i-Butenyl-, Pentyl-, Pentenyl-, Pentinyl-, Hexyl-, Hexenyl-, Heptyl-, Heptenyl-, Octyl-, Octenyl-, Nonyl-, Nonenyl-, Decyl-, Decenyl-, Dodecyl- oder Dodecenylreste. Die Alkyl-, Alkenyl- oder Alkinylreste können noch unter den Reaktionsbedingungen inerte Substituenten tragen, z.B. Halogen, Alkoxy-oder Carboxygruppen.

Cycloalkylreste sind z.B. Cyclopentyl-, Cyclohexyl-, Cycloheptyl-, Cyclopentenyl- oder Cyclohexenylreste.

Als aromatische Reste kommen z.B. Phenyl-, Benzyl-, 2-Phenylethyl-, 3-Phenylpropyl-, 2-Phenylpropyl-,

4-Phenylbutyl-, 3-Phenylbutyl-, 2-Phenylbutyl- oder 3-Phenylbutenyl-Reste in Betracht, die gegebenenfalls noch durch unter den Reaktionsbedingungen inerte Reste substituiert sein können.

Heterocyclische bzw. heteroaromatische Reste sind z.B. Tetrahydrofuran-, Dihydrofuran-, Furan-, Tetrahydrothiophen(Thiophan)-, Dihydrothiophen-, Thiophen-, Pyridin-, Thiopyran. Diese Reste können noch durch unter den Reaktionsbedingungen inerte Reste, wie Alkylreste oder Halogenatome substituiert sein.

Für das erfindungsgemäße Verfahren besonders geeignete Ausgangsstoffe sind solche 1,3-Dioxane, in denen $R^3$ Wasserstoff bedeutet, während $R^4$ und $R^5$ einen der genannten organischen Reste darstellen.

Die Ausgangsverbindungen der Formel (II) lassen sich nach bekannten Methoden aus Aldehyden oder Ketonen oder deren leicht spaltbaren Derivaten, z.B. Dialkylketalen oder Acetalen mit 1,3-Diolen gemäß folgender Reaktionsgleichung herstellen.

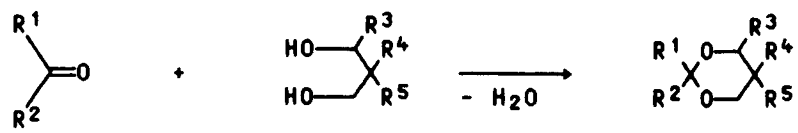


Als Diolkomponente kommen z.B. folgende Verbindungen in Betracht: Propandiol-1,3, 2-Methyl-, 2-Ethyl-, 2-Phenyl-, 2,2-Dimethyl-, 2,2-Diethyl-, 2-Methyl-2-ethyl-, 2-Methyl-2-propyl-, 2-Methyl-2-butyl-, 2-Methyl-2-phenyl und 2-Ethyl-2-butyl-propandiol-1,3, 1,1-Dimethylolcyclohexan und -pentan, 3,3-Dimethyloltetrahydrofuran und -pyran sowie 2,2,4-Trimethylpentadiol-1,3.

Geeignete Carbonylkomponenten sind z.B. aliphatische, aromatische oder heterocyclische Aldehyde und Ketone oder deren Acetale bzw. Ketale mit niedrig siedenden Alkoholen.

Gesättigte aliphatische Aldehyde sind z.B. Form-, Acet-, Propion- oder Butyraldehyd, Pentanal, Hexanal und höhere homologe n-Alkanale, wie Dekanal, Isobutyraldehyd, 2-Methylbutanal, 3-Methylbutanal, 3,3-Dimethylbutanal, 2-Methylpentanal, 2-Ethylhexanal und 2-Methyldekanal, Glyoxal, Methylglyoxal, Malondialdehyd, Succindialdehyd und Glutardialdehyd.

Heterocyclische Aldehyde sind z.B. Tetrahydrofuryl-2-aldehyd und -3-aldehyd, Tetrahydrothienyl-2- und -3-aldehyd, 5,6-Dihydropyranyl-6-aldehyd, 2,5-Dimethyl-5,6-dihydropyranyl-6-aldehyd, Furyl-2-aldehyd und -3-aldehyd. Thienyl-3-aldehyd, 2-, 3- oder 4-Pyridylaldedhyd.

Als Ketone kommen beispielsweise die folgenden Verbindungen in Betracht: Aceton, Methylethylketon, Diethylketon, Methylisopropylketon, Diisopropyl- und Diisobutylketon, Methylisobutylketon, Methoxyaceton, Methylvinylketon, Methylisopropenyl- und Methylisobutenylketon, Cyclopentanon, Cyclohexanon, Methylcyclopentanone, Methylcyclohexanone, Cyclohexenon, 3,5,5-Trimethylcyclohexenon-2, Methyl-, Ethyl- und Vinylphenylketon, Methylfurylketon, Acetylaceton und Acetessigester.

Weitere substituierte Alkanale sind beispielsweise 3-Hydroxy-2,2-dimethylpropanal, Methoxy- und Butoxypivalinaldehyd, 4-Acetoxybutyraldehyd und 5-Formylvaleriansäureethylester.

Weiterhin können ungesättigte Aldehyde verwendet werden, z.B. Acrolein, $\alpha$-Methylacrolein, $\alpha$-Ethylacrolein und höhere $\alpha$-Alkyl-, Isoalkyl und Alkenylacroleine, wie But-2-enal, 2-Methyl-but-2-enal, 2-Methylpent-2-enal, 2-Ethyl-hex-2-enal, 2,2-Dimethyl-pent-4-enal, 2-Methyl-4-acetoxy-but-2-enal, 2-Methoxymethylacrolein, 2-(3-Methoxycarbonyl-propyl-)-acrolein oder 2-Methyl-4-chlor-but-2-enal.

Aromatische Aldehyde sind z.B. Benzaldehyd, p-Methoxybenzaldehyd, Phenylacetaldehyd, 2-Phenyl- und 3-Phenylpropanal, 2-Hydroxybenzaldehyd, 3-Hydroxy-4-methoxybenzaldehyd, Zimtaldehyd oder Benzylacrolein.

Als Katalysatoren für die erfindungsgemäße Umwandlung von 1,3-Dioxanen werden Metalloxid-Katalysatoren ohne Zeolithstruktur, die mit anorganischen Säuren behandelt worden sind, eingesetzt.

Geeignete Metalloxid-Katalysatoren sind z.B. die sauer wirkenden Oxide von Elementen der III. und IV. Hauptgruppe sowie der IV. bis VI. Nebengruppe des periodischen Systems, insbesondere Oxide wie Siliciumdioxid in Form von Kieselgel, Kieselgur, Quarz, weiterhin Titandioxid, Zirkondioxid, Phosphoroxide, Vanadiumpentoxid, Nioboxid, Bortrioxid, Aluminiumoxid,, Chromoxide, Molybdänoxide, Wolframoxide, Eisenoxide oder Gemische dieser Oxide.

Als anorganische Säuren können z. B. HF, HCl, HBr, HJ, $H_2SO_4$, $H_2SO_3$, $HNO_3$, $H_3BO_3$, Phosphorsäuren oder deren Gemische verwendet werden.

Die Behandlung der Metalloxide, z. B. des $SiO_2$ (Silica), mit Säuren kann sowohl am verformten als auch unverformten Material vorgenommen werden. Dabei geht man vorteilhaft z. B. so vor, daß man $SiO_2$ in Pulverform mit 1 n Säure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise

behandelt man z. B. $SiO_2$ vor oder nach ihrer Verformung 1 bis 3 Stunden bei Temperaturen von 60 bis 80 °C mit einer 3 bis 25 gew.%igen, insbesondere 12 bis 20 gew.%igen wäßrigen Salzsäure. Anschließend wird das so behandelte $SiO_2$ mit Wasser gewaschen, getrocknet und bei 400 bis 500 °C calciniert.

Eine besondere Ausführungsform für die Säurebehandlung besteht darin, daß man das $SiO_2$ vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z. B. durch Abfiltrieren und Auswaschen des Materials wird dieses zweckmäßig bei Temperaturen von 100 bis 160 °C getrocknet und bei Temperaturen von 450 bis 600 °C calciniert. Gemäß einer weiteren bevorzugten Ausführungsform für die Säurebehandlung wird das $SiO_2$ nach seiner Verformung bei erhöhter Temperatur, z. B. von 50 °C bis 90 °C, vorzugsweise 60 bis 80 °C, über einen Zeitraum von 0,5 bis 5, vorzugsweise 1 bis 3 Stunden mit 12 bis 20 gew.%iger Salzsäure, behandelt. Anschließend wird dieses Material ausgewaschen und bei Temperaturen von 100 bis 160 °C, getrocknet und bei Temperaturen von 450 bis 600 °C calciniert. Einer HF-Behandlung kann sich auch eine HCl-Behandlung anschließen.

Auch mit Phosphorsäuren behandelte Katalysatoren können eingesetzt werden. Phosphorsäure wird z.B. auf $SiO_2$-, $Al_2O_3$- oder $TiO_2$-Träger durch Auftränken oder Versprühen aufgebracht. Ein phosphorsäurehaltiger Katalysator kann beispielsweise durch Behandlung von $H_3PO_4$- oder $NaH_2PO_4$-Lösung auf $SiO_2$ und anschließende Trocknung bzw. Calcination erhalten werden. Phosphorsäure kann aber auch mit Kieselgel zusammen in einem Sprühturm versprüht werden. Danach schließt sich eine Trocknung und meist eine Calcination an. Phosphorsäure kann auch in einer Imprägniermühle auf das Trägermaterial aufgesprüht werden.

Durch partielle Verkokung (pre-coke) ist es möglich, die Aktivität des Katalysators für eine Selektivitätsoptimum des gewünschten Reaktionsproduktes einzustellen.

Die hier beschriebenen Katalysatoren können wahlweise als 2- bis 4-mm-Stränge oder als Tabletten mit 3 bis 5 mm Durchmesser oder als Pulver mit Teilchengrößen von 0,1 bis 0,5 mm oder als Wirbelkontakt eingesetzt werden.

Die für die erfindungsgemäße Umwandlung gewählten Reaktionsbedingungen sind in der bevorzugten Gasphase 200 bis 500 °C, vorzugsweise 230 bis 400 °C und eine Belastung WHSV = 0,1 bis 20 $h^{-1}$, bevorzugt 0,5 bis 5 $h^{-1}$ (g 1,3-Dioxan/g Katalysator und Stunde). Die Reaktion kann im Festbett oder Wirbelbett durchgeführt werden. Es ist auch möglich, die Reaktion in der Flüssigphase, in Suspensions-, Riesel- oder Sumpffahrweise bei Temperaturen zwischen 50 und 200 °C durchführen. Im allgemeinen steigt der Umsatz mit steigender Temperatur stark an, während die Selektivität in einen bestimmten Temperaturbereich nur wenig zurückgeht.

Das Verfahren kann bei Normaldruck oder je nach Flüssigkeit der Ausgangsverbindung bei vermindertem oder erhöhtem Druck durchgeführt, wobei die Durchführung vorzugsweise kontinuierlich erfolgt.

Schwerflüchtige oder feste Ausgangsstoffe kann man in gelöster Form, z. B. in THF-, Toluol- oder Petrolether-Lösung einsetzen. Es ist auch eine Verdünnung des Ausgangsstoffes mit derartigen Lösungsmitteln oder mit Inertgasen wie $N_2$, Ar, $H_2O$-Dampf möglich.

Nach der Umsetzung werden die entsprechenden 4-Oxa-aldehyde nach den dafür üblichen Techniken z. B. durch Destillation aus dem Reaktionsgemisch isoliert; nichtumgesetzte 1,3-Dioxane (II) werden gegebenenfalls für die erfindungsgemäße Umsetzung zurückgeführt.

Die nach dem erfindungsgemäßen Verfahren zugänglichen Verbindungen und eine Reihe ihrer Derivate sind als biologisch aktive Verbindungen, z. B. als Bakterizide, von Interesse und auch wertvolle Zwischenprodukte. Sie lassen sich nach geläufigen Methoden, in einfacher Weise zu Aminen, Alkoholen und Säuren z. B. durch Oxidation mit Sauerstoff oder durch Reduktion z. B. durch katalytische Hydrierung oder aminierende Hydrierung weiterverarbeiten.

Die neue Verbindung der Formel

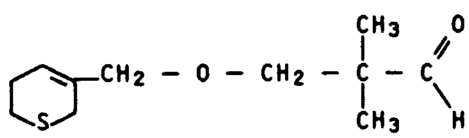

hat einen Smp. 53,8 °C und einen Sdp. von 121 bis 125 °C.

Beispiele 1 bis 34

Die Reaktionen werden unter isothermen Bedingungen in einem Rohrreaktor (Wendel, 0,6 cm Innendurchmesser, 90 cm Länge) in der Gasphase mindestens 6 Stunden lang durchgeführt. Die Trennung und Charakterisierung der Reaktionsprodukte erfolgte nach üblichen Methoden. Die quantitative Bestimmung der Reaktionsprodukte (I) und der Ausgangsstoffe (II) erfolgte gaschromatographisch.

Die in den Beispielen verwendeten Katalysatoren für die Umwandlung von 1,3-Dioxanen zu 4-Oxaaldehyden sind:

Katalysator A

100 g $SiO_2$-Stränge (D 11 - 10®) werden mit 600 ml 15%iger HCl bei 80 ° C 1 h behandelt. Danach wird das Material Cl-frei gewaschen, bei 110 ° C getrocknet und bei 600 ° C/1 h calciniert.

Katalysator B

100 g $SiO_2$-Stränge (D 11 - 10) werden 280 ml 0,1 n HF und 80 ml $H_2O$ unter Rückfluß 1 h behandelt. Danach wird das Material neutral gewaschen, bei 110 ° C getrocknet und bei 500 ° C/5 h calciniert.

Katalysator C

50 g des Katalysators B werden in 300 ml 15%iger HCl bei 80 ° C 1 h behandelt. Danach wird das Material Cl-frei gewaschen, bei 110 ° C getrocknet und bei 600 ° C 1 h calciniert.

Katalysator F

$Al_2O_3$ (D 10 - 10®) wird mit $H_3BO_3$ imprägniert, bei 130 ° C/2 h getrocknet und bei 540 ° C/2 h calciniert. Der Katalysator F setzt sich zusammen aus 85 % $Al_2O_3$ und 15 % $B_2O_3$.

Katalysator G

Katalysator G wird erhalten, indem man D 10 - 10 $Al_2O_3$ mit 85 %iger $H_3PO_4$ 30 min behandelt, danach 130 ° C/2 h trocknet und bei 540 ° C/2 h calciniert. Der P-Gehalt beträgt 4,9 Gew.%.

Katalysator H

D 11 - 10 $SiO_2$ wird mit $H_3BO_3$ in methanolischer Lösung behandelt, bei 110 ° C getrocknet und bei 500 ° C/5 h calciniert. Der $B_2O_3$-Gehalt des Katalysators H beträgt 3,0 Gew.%.

Katalysator I (Vergleichskatalysator)

D 11 10 $SiO_2$.

Katalysator J (Vergleichskatalysator)

D 10 - 10 $Al_2O_3$.

Katalysator K (Vergleichskatalysator)

$TiO_2$ P 25® wird zu 2-mm-Strängen verformt, bei 110 ° C getrocknet und bei 500 ° C/16 h calciniert.

Katalysator L (Vergleichskatalysator)

Handelsübliches MgO in Tablettenform.

Katalysator M

Katalysator M wird erhalten, indem man Katalysator L, wie bei Katalysator B beschrieben, behandelt.

Katalysator O (Vergleichskatalysator)

Katalysator O wird nach DOS 29 22 698 hergestellt. Hierbei werden 51 g eines handelsüblichen $SiO_2$ D 11 - 11®) mit Lösung aus 51 g 0,1 n $CH_3COOH$, 3,19 g $Pr(NO_3)_3 \times 5 H_2O$, 3,21 g $Nd(NO_3)_3 \times 5 H_2O$ und 2,66 g $CH_3COOK$ imprägniert, getrocknet und bei 600 ° C/4 h calciniert.

Die mit diesen Katalysatoren erzielten Versuchsergebnisse sind in den nachfolgenden Tabellen 1 und 2 aufgeführt.

**Tabelle I**

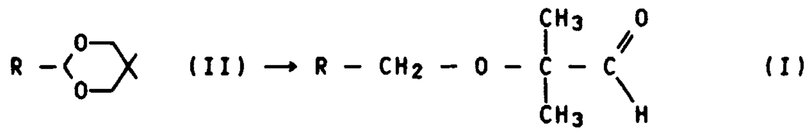

| Beispiel | 1 | 2 | 3 | 4 1) | 5 1) | 6 1) | 7 1) |
|---|---|---|---|---|---|---|---|
| R | | | | $CH_3CH_2CH_2$- | | | |
| Katalysator | A | F | M | J | K | L | O |
| Temp. °C | 300 | 300 | 300 | 300 | 300 | 300 | 300 |
| WHSV $h^{-1}$ | 3 | 3 | 3 | 3 | 3 | 3 | 3 |
| Umsatz II, % | 74,5 | 53,3 | 23,3 | 22,2 | 5,0 | 5,6 | 28,5 |
| Selekt. I, % | 87,7 | 79,2 | 75,2 | 71,6 | 26,0 | 5 | 13,2 |

1) Vergleichsbeispiele

Das Vergleichsbeispiel 7 zeigt, daß der in der DE-OS 29 22 698 beschriebene Katalysator O nur bei kurzen Reaktionszeiten von 1 bis 2 Stunden wirksam ist, während nach 4 Stunden die Aktivität praktisch auf Null abgesunken ist.

**Tabelle 1 (Fortsetzung)**

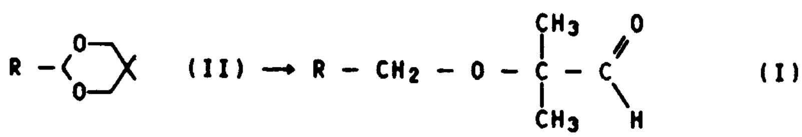

| Beispiel | 8 | 9 | 10 | 11 | 12 | 13 | 14 | 15 | 16 |
|---|---|---|---|---|---|---|---|---|---|
| R | 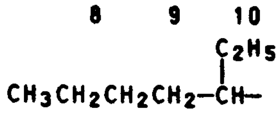 | | | | 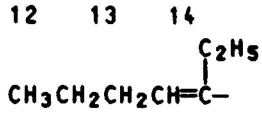 | | | 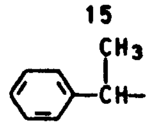 | 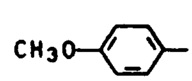 |
| Katalysator | A | A | C | | I 1) | A 2) | | A | A |
| Temp. °C | 250 | 300 | 300 | | 300 | 250 | | 300 | 300 |
| WHSV $h^{-1}$ | 2 | 2 | 2 | | 2 | 2 | | 2 | 2 |
| Umsatz II,% | 86,9 | 94,6 | 93,1, | 8 | 96,6 | 84,3 | | 89,3 | 100 |
| Selekt. I,% | 84,8 | 53,8 | 64,3, | 7 | 29,2 | 79,5 | | 85,8 | 67,9 |

1) Vergleichsbeispiel

2) verdünnt mitTHF; Acetal : THF = 50 : 50

Tabelle 1 (Fortsetzung)

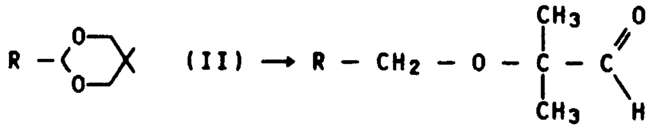
(II) (I)

| Beispiel | 17 | 18 | 19 | 20 | 21 |
|---|---|---|---|---|---|
| R | | | 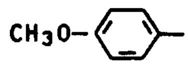 | | |
| Katalysator | | G | H | F | I[1)] |
| Temp. °C | | 300 | 300 | 300 | 300 |
| WHSV $h^{-1}$ | | 2 | 2 | 3 | 2 |
| Umsatz II, % | | 39,4 | 95,3 | 40,8 | 72,0 |
| Selekt. I, % | | 86,0 | 62,8 | 81,4 | 67,9 |

1) Vergleichsbeispiel

Tabelle 2

| Beispiel | 22 | 23 | 24 | 25 | 26 | 27 | 28 |
|---|---|---|---|---|---|---|---|
| Reaktion | A.) | A.) | B.) | B.) | B.) | B.) | C.) |
| Katalysator | A | B | A | A | B | C | A |
| Temp. °C | 250 | 300 | 250 | 300 | 300 | 300 | 300 |
| WHSV $h^{-1}$ | 2 | 2 | 2 | 2 | 2 | 2 | 2,5 |
| Umsatz II, % | 94,1 | 97,3 | 18,2 | 78,1 | 56,6 | 75,1 | 10,9 |
| Selekt. I, % | 69,9 | 51,8 | 95,6 | 79,5 | 90,6 | 73,5 | 71,6 |

Reaktionen:

A.) 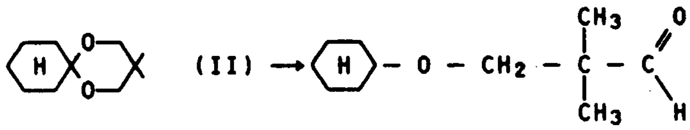 (II) → (I)

B.) 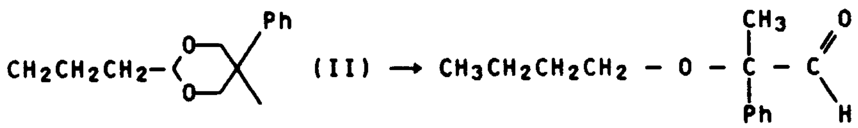 (II) → (I)

Ph = Phenyl

C.) 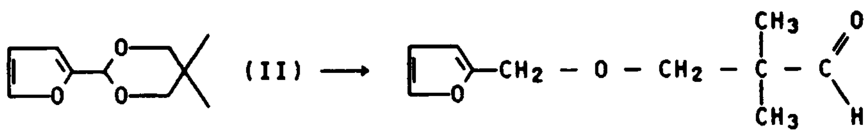 (II) → (I)

D.) 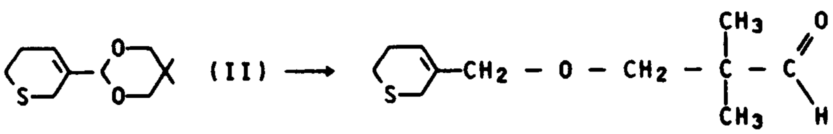 (II) → (II)

mit THF 50 : 50 verdünnt

## Patentansprüche

1. Verfahren zur Herstellung von 4-Oxa-aldehyden der Formel (I)

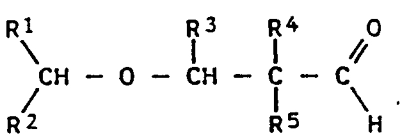

(I)

durch katalytische Isomerisierung von 1,3-Dioxanen, dadurch gekennzeichnet, daß man 1,3-Dioxane der Formel (II)

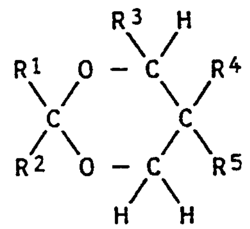

(II),

wobei die Reste $R^1$, $R^2$, $R^4$ und $R^5$ in den Formeln (I) und (II) untereinander gleich oder verschieden sind und für Wasserstoff, geradkettige oder verzweigte Alkyl-, Alkenyl oder Alkinylreste mit bis zu 18 Kohlenstoffatomen, Cycloalkyl- oder Cycloalkenylreste mit 5 bis 8 Kohlenstoffatomen, Aryl-, Alkylaryl-, Aralkyl-, Aralkenyl- oder Alkenylarylreste mit 5 bis 16 Kohlenstoffatomen oder für heterocyclische Reste stehen und darüber hinaus jeweils die Reste $R^1$ und $R^2$ und/oder $R^4$ und $R^5$ zusammen mit dem C-Atom, an das sie gebunden sind, ein Cycloalkan, Cycloalken oder einen Heterocyclus bilden können, wobei die genannten Reste noch unter den Reaktionsbedingungen inerte Substituenten tragen können und wobei der Rest $R^3$ Wasserstoff oder einen geradkettigen oder verzweigten Alkylrest bedeutet, unter Verwendung von Metalloxid-Katalysatoren ohne Zeolithstruktur, die mit anorganischen Säuren behandelt worden sind, isomerisiert.

**2.** Verfahren nach Anspruch 1, dadurch gekennzeichnet, daß Acetale oder Ketale des Propandiols-1,3, des 2-Methyl-, 2,2-Dimethyl-, 2-Methyl-2-Ethyl-, 2-Methyl-2-propyl-, 2-Methyl-2-butyl-, 2-Hethyl-2-phenyl-, 1-Isopropyl-2,2-dimethyl-propandiols-1,3 oder des 1,1-Dimethylolcyclohexans oder -pentans isomerisiert werden.

**3.** Verfahren nach Anspruch 1 und 2, dadurch gekennzeichnet, daß man als Katalysatoren Metalloxide der Elemente Si, Al, Ti, Zr, Ce ohne Zeolithstruktur, die mit HCl und/oder HF und/oder $H_3PO_4$ und/oder $H_3BO_3$ behandelt worden sind, oder deren Gemische verwendet.

**4.** Verfahren nach den Ansprüchen 1 bis 4, dadurch gekennzeichnet, daß man die Umsetzung in der Gasphase durchführt.

## Claims

**1.** A process for the preparation of a 4-oxaaldehyde of the formula (I)

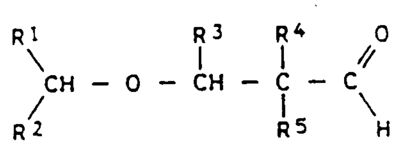

(I)

by catalytic isomerization of a 1,3-dioxane, wherein a 1,3-dioxane of the formula (II)

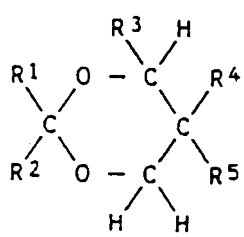

(II)

where $R^1$, $R^2$, $R^4$ and $R^5$ in the formulae (I) and (II) are identical or different and are each hydrogen, a straight-chain or branched alkyl, alkenyl or alkynyl radical of not more than 18 carbon atoms, a

cycloalkyl or cycloalkenyl radical of 5 to 8 carbon atoms, an aryl, alkylaryl, aralkyl, aralkenyl or alkenylaryl radical of 5 to 16 carbon atoms or a heterocyclic radical and moreover $R^1$ and $R^2$ and/or $R^4$ and $R^5$ together with the carbon atom to which they are bonded may form a cycloalkane, cycloalkene or a heterocyclic structure, and the stated radicals may furthermore carry substituents which are inert under the reaction conditions, and $R^3$ is hydrogen or straight-chain or branched alkyl, is isomerized using a metal oxide catalyst, which does not have a zeolite structure and has been treated with an inorganic acid.

**2.** A process as claimed in claim 1, wherein an acetal or ketal of propane-1,3-diol, of 2-methyl-, 2,2-dimethyl-, 2-methyl-2-ethyl-, 2-methyl-2-propyl, 2-methyl-2-butyl-, 2-methyl-2-phenyl- or 1-isopropyl-2,2-dimethylpropane-1,3-diol or of 1,1-dimethylolcyclohexane or -pentane is isomerized.

**3.** A process as claimed in claim 1 or 2, wherein the catalyst used is a metal oxide of the elements Si, Al, Ti, Zr or Ce, which does not have a zeolite structure and has been treated with HCl and/or HF and/or $H_3PO_4$ and/or $H_3BO_3$, or a mixture of these.

**4.** A process as claimed in any of claims 1 to 4, wherein the reaction is carried out in the gas phase.

**Revendications**

**1.** Procédé de préparation de 4-oxa-aldéhydes de formule (I)

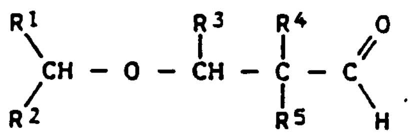

(I)

par isomérisation catalytique de 1,3-dioxannes, caractérisé en ce qu'an isomérise des 1,3-dioxannes de formule (II)

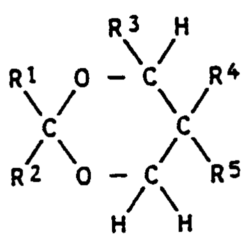

(II),

les restes $R^1$, $R^2$, $R^4$ et $R^5$ dans les formules (I) et (II) étant mutuellement identiques au différents et étant des atomes d'hydrogène, des restes alkyle, alcényle ou alcynyle à chaîne droite ou ramifiée renfermant jusqu'à 18 atomes de carbone, des restes cycloalkyle ou cycloalcényle à 5-8 atomes de carbone, des restes aryle, alkylaryle, aralkyle, aralcényle ou alcénylaryle à 5-16 atomes de carbone au des restes hétérocycliques, et en outre les restes $R^1$ et $R^2$ et/ou $R^4$ et $R^5$ pouvant chaque fois former ensemble, avec l'atome de carbone auquel ils sont fixés, un cycloalcane, cycloalcène ou un hétérocycle, les restes mentionnés pouvant encore porter des substituants inertes dans les conditions de la réaction, et le reste $R^3$ étant un atome d'hydrogène ou un reste alkyle à chaîne droite ou ramifiée, en utilisant des catalyseurs à base d'oxydes métalliques sans structure zéolithique, qui ont été traités par des acides minéraux.

**2.** Procédé selon la revendication 1, caractérisé en ce que des acétals au des cétals du propanediol-1,3, du 2-méthyl-, 2,2-diméthyl-, 2-méthyl-2-éthyl-, 2-méthyl-2-propyl-, 2-méthyl-2-butyl-, 2-methyl-2-phényl-, 1-isopropyl-2,2-diméthyl-propanediol-1,3 ou du 1,1-diméthylolcyclohexane ou -pentane sont isomérisés.

3. Procédé selon la revendication 1 au 2, caractérisé en ce qu'on utilise, comme catalyseurs, des oxydes métalliques des éléments Si, Al, Ti, Zr, Ce sans structure zéolithique, qui ont été traités par HCl et/ou HF et/ou $H_3PO_4$ et/ou $H_3BO_3$.

4. Procédé selon l'une quelconque des revendications 1 à 3, caractérisé en ce qu'on mène la réaction en phase gazeuse.